# FASCICULE DE BREVET EUROPEEN

(11) **EP 1 131 287 B1**
(45) Date de publication et mention de la délivrance du brevet: **09.04.2003**
(21) Numéro de dépôt: 99956070.9
(22) Date de dépôt: 18.11.1999
(51) Int. Cl.: C07D 201/16, C07D 201/08

(54) **PROCEDE DE TRAITEMENT DE LACTAMES ET PROCEDE DE PURIFICATION D'UN LACTAME**
VERFAHREN ZUR BEHANDLUNG VON LAKTAMEN UND VERFAHREN ZUR LAKTAMREINIGUNG
METHOD FOR TREATING LACTAMS AND METHOD FOR PURIFYING A LACTAM

(30) Priorité: 19.11.1998 FR 9814735
(43) Date de publication de la demande: 12.09.2001
(73) Titulaire: Rhodia Polyamide Intermediates, 69192 Saint-Fons (FR)
(72) Inventeur: LECONTE, Philippe, F-69330 Meyzieu (FR)
(74) Mandataire: Esson, Jean-Pierre
(86) Numéro de dépôt international: FR9902823
(87) Numéro de publication internationale: WO00031031

(56) Documents cités:
- EP-A- 0 264 126
- FR-A- 2 235 121
- FR-A- 2 351 959
- US-A- 5 496 941
- CHEMICAL ABSTRACTS, vol. 79, no. 12, 24 septembre 1973 (1973-09-24) Columbus, Ohio, US; abstract no. 67052, KAWAMOTO I. ET AL: "Purification of epsilon-caprolactam" XP002110353 & JP 48 015955 A (TORAY INDUSTRIES, INC.)
- CHEMICAL ABSTRACTS, vol. 116, no. 6, 10 février 1992 (1992-02-10) Columbus, Ohio, US; abstract no. 42203, WINZER W. ET AL: "Purging impurities from the extraction loop in caprolactam recovery" XP002110354 & DD 293 580 A (LEUNA-WERKE A.G.)
- CHEMICAL ABSTRACTS, vol. 86, no. 18, 2 mai 1977 (1977-05-02) Columbus, Ohio, US; abstract no. 122008, TAKEUCHI J. ET AL: "Epsilon-caprolactam" XP002110355 & JP 51 138690 A (UBE INDUSTRIES, LTD.)
- CHEMICAL ABSTRACTS, vol. 86, no. 14, 4 avril 1977 (1977-04-04) Columbus, Ohio, US; abstract no. 90566, TAKEUCHI J. ET AL: "Epsilon-caprolactam recovery" XP002110356 & JP 51 128992 A (UBE INDUSTRIES, LTD.)
- CHEMICAL ABSTRACTS, vol. 78, no. 2, 15 janvier 1973 (1973-01-15) Columbus, Ohio, US; abstract no. 4752, OYAMA F. ET AL: "Epsilon-caprolactam" XP002110357 & JP 47 020182 A (SUMITOMO CHEMICAL CO., LTD.)

## Description

La présente invention concerne un procédé de traitement de lactames.

Elle concerne plus particulièrement un procédé permettant de modifier la nature chimique des impuretés contenues dans un milieu contenant de l'^{ε}-caprolactame soit pour les transformer en composés non gênants soit en composés extractibles dans des opérations de purification ultérieures.

Les lactames et notamment l'^{ε}-caprolactame, monomère pour la préparation du polycaproamide (PA6) sont préparés selon plusieurs procédés de synthèse.

Le procédé le plus utilisé industriellement met en oeuvre une réaction de réarrangement de Beckmann de l'oxime de la cyclohexanone avec de l'acide sulfurique ou de l'oléum, suivie de la neutralisation du milieu par l'ammoniac, puis de la séparation et de la purification du lactame.

Un autre procédé proposé de synthèse du lactame consiste à faire une hydrolyse cyclisante d'un aminoalkylnitrile comme le 6-aminocapronitrile pour la préparation de l'^{ε}-caprolactame. Cette réaction peut être réalisée en présence ou en absence d'un catalyseur, en phase liquide ou vapeur. Cette réaction libère de l'ammoniac.

Ce dernier procédé est décrit dans de nombreux brevets. A titre d'exemple, on peut citer les brevets US 2 357 484, US 2 301 964, FR 2 029 540.

Le procédé de préparation de lactames par hydrolyse cyclisante en phase vapeur d'aminonitriles est également décrit dans les demandes de brevet EP 0 659 741 et WO 96/22974.

La conversion d'aminocapronitrile en ^{ε}-caprolactame en présence d'eau est exemplifiée dans les brevets US 2 245 129 et EP 0 150 295.

Généralement dans ces derniers procédés, l'aminocapronitrile est obtenu par hémihydrogénation de l'adiponitrile par des procédés connus et décrits notamment dans les brevets DE 836 938; DE 848 654 ou US 5 151 543.

Comme l'utilisation principale des lactames produits est la fabrication de polymères ou copolymères et plus particulièrement de polyamides ou copolyamides destinés à être mis en forme pour la production de fils, fibres, pièces moulées ou films, la pureté des lactames doit respecter des spécifications précises et drastiques.

Ainsi, une des spécifications importantes est l'absorbance UV d'une solution aqueuse de caprolactame représentés par un indice UV. Cet indice est déterminé par mesure de l'absorbance d'une solution aqueuse de caprolactame (50 % en poids) à la longueur d'onde de 290 dans une cellule de 1 cm de large.

Pour obtenir un indice UV faible, il est connu, notamment de soumettre le lactame à une hydrogénation en présence de catalyseur.

Ainsi, le brevet allemand 1 253 716 décrit l'hydrogénation du caprolactame obtenu par le procédé de réarrangement de Beckmann en présence de catalyseur d'hydrogénation en suspension.

De même, le brevet DE 1 004 616 et est-allemand 75 083 décrit un procédé d'hydrogénation du caprolactame après traitement avec le charbon actif et des résines échangeuses d'ions.

Le brevet US 5 496 941 décrit un procédé de purification du lactame obtenu par hydrolyse cyclisante d'un aminonitrile. Ce procédé comprend une étape d'hydrogénation du lactame en présence d'un catalyseur d'hydrogénation. Un solvant tel que l'eau ou un alcool est préférablement présent.

L'étape d'hydrogénation est réalisée sur le lactame isolé et séparé du milieu réactionnel d'hydrolyse cyclisante. En effet, ce procédé requiert la séparation des composés plus volatiles et moins volatiles que le caprolactame du milieu d'hydrolyse cyclisante. Cette séparation requiert donc l'élimination de l'ammoniac produit et la distillation du caprolactame.

Ces différents procédés donnent des résultats satisfaisants pour l'obtention d'un lactame à indice UV faible. Toutefois, cette étape de purification est pénalisante pour l'économie générale du procédé. En effet, l'efficacité du catalyseur diminue très rapidement. Cette durée de cycle court du catalyseur requiert un remplacement fréquent de celui-ci, et entraîne un risque d'obtenir un lactame de pureté insatisfaisante en fin de cycle du catalyseur. Ce procédé est plus pénalisant encore quand le catalyseur ne peut être régénéré par un procédé simple et économique.

Un des buts de la présente invention est notamment de remédier à ces inconvénients en proposant des conditions de mise en oeuvre de l'étape d'hydrogénation permettant d'augmenter considérablement au moins la durée de cycle des catalyseurs d'hydrogénation et donc l'économie générale du procédé de fabrication de lactames à pureté élevée.

A cet effet, l'invention à pour objet un procédé de traitement d'un milieu liquide comprenant au moins de l'^{ε}, notamment pour diminuer l'indice UV du lactame, consistant à soumettre ledit milieu à une hydrogénation en présence d'un catalyseur d'hydrogénation. Le procédé se caractérise par la présence d'ammoniac dissous dans ledit milieu.

La présence d'ammoniac permet d'augmenter de manière importante la durée de cycle du catalyseur d'hydrogénation.

Pour éviter ou limiter la réaction de condensation du lactame, la température de mise en oeuvre de l'hydrogénation sera réalisée à une température permettant d'avoir une cinétique d'hydrogénation compatible avec une exploitation industrielle, mais la plus basse possible. De préférence, cette température est inférieure à 150°C, notamment quand le lactame est l'^{ε}-caprolactame. Avantageusement, la température est comprise entre 50°C et 150°C, préférentiellement entre 70°C et 130°C.

La concentration en ammoniac dans le milieu peut varier dans de grandes proportions, mais est avantageusement supérieure à 10 g/l, de préférence comprise entre 50 g/l et 200 g/l.

Le milieu contenant le lactame comprend, selon une caractéristique de l'invention, un solvant choisi par exemple dans les alcools comprenant de 1 à 3 atomes de carbone. Toutefois, les solvants préférés de l'invention sont l'eau et les mélanges eau / alcool.
En outre, il est possible de réaliser l'hydrogénation de l'^{ε}-caprolactame fondu, sans solvant autre que l'ammoniac.

Le procédé de traitement de l'invention est réalisé en présence d'un catalyseur d'hydrogénation. Ce catalyseur peut être mis en suspension dans le milieu, ou présent sous la forme d'un lit fixe ou lit fluidisé déposé dans un réacteur tubulaire. Le catalyseur peut être un catalyseur massique ou supporté.

Les catalyseurs préférés de l'invention sont ceux dérivés d'un ou plusieurs métaux choisis dans le groupe comprenant le fer, le nickel, le cobalt, le ruthénium, le rhodium, le palladium, l'osmium, l'iridium, le platine.

Comme support de catalyseur on peut utiliser par exemple, le charbon actif, les alumines, les silices, les oxydes de titane, les oxydes de terres rares comme les oxydes de lanthane ou de cérium, les oxydes de zirconium ou de zinc. On peut également utiliser un mélange de ces oxydes ou des oxydes mixtes. On peut également utiliser comme support de catalyseur des silicates ou phosphates de magnésium, aluminium, bore.

Dans le cas de catalyseur supporté, la concentration en élément catalytique exprimé en poids de métal est avantageusement compris entre 0,01 et 80 % du poids total du catalyseur, préférentiellement de 0,1 à 50 % en poids.

Par ailleurs, les catalyseurs peuvent comprendre des additifs améliorant l'effet catalytique tels que par exemple du zirconium, du manganèse, du cuivre, du chrome, du titane, du molybdène, du tungstène, du fer ou du zinc.

Ces éléments dopants représentent habituellement en poids par rapport au métal catalytiquement actif de 0 à 15 % et de préférence de 0,1 à 10 %.

La fabrication de ces catalyseurs supportés ou non est décrite dans de nombreux documents tels que dans l'encyclopédie ULLMANN de l'Industrie Chimique, volume A5, pages 348 - 350, 5ème édition.

Le traitement d'hydrogénation est réalisé soit à pression atmosphérique, soit à une pression comprise entre **1** et 100 bars.

Comme cela est décrit dans les documents cités, ce traitement par l'hydrogène permet de diminuer notamment l'indice UV du caprolactame. Mais, la présence d'ammoniac dissous permet d'obtenir un indice UV faible même après une durée longue de fonctionnement en continu de l'opération, c'est-à-dire de traiter une quantité importante de lactame sans avoir à remplacer ou régénérer le catalyseur présent dans le réacteur, ou en minnimisant la consommation de catalyseur consommé par kilogramme de lactame traité.

L'invention s'applique au traitement de l'^{ε}-caprolactame de diffférentes origines tels que ceux obtenus par la réction d'arrangement de Beckmann, l'^{ε}-caprolactame obtenu par dépolymérisation de polyamide ou encore l'^{ε}-caprolactame obtenu par hydrolyse cyclisante de l'aminocapronitile, par exemple.

Toutefois, l'invention s'applique plus particulièrement au traitement des solutions de lactames obtenues par hydrolyse d'un aminonitrile soit en phase vapeur, soit en phase liquide.

Ainsi, le procédé de l'invention s'applique plus particulièrement, dans les étapes d'hydrogénation décrites dans les procédés de purification de caprolactame obtenu par hydrolyse cyclisante d'un aminonitrile en phase vapeur ou en phase liquide décrit dans le brevet US 5 496 941 et la demande de brevet WO 98/05636.

Un autre objet de l'invention consiste en un procédé de purification de lactame obtenu par hydrolyse cyclisante d'un aminonitrile en phase vapeur et plus particulièrement un procédé de purification d'^{ε}-caprolactame obtenu par hydrolyse cyclisante en phase vapeur du 6-aminocapronitrile.

Ce procédé consiste :
- à refroidir le milieu réactionnel d'hydrolyse à une température inférieure à 150°C,
- à soumettre le milieu réactionnel refroidi contenant au moins partiellement l'ammoniac formé, à une hydrogénation en présence d'un catalyseur d'hydrogénation,
- après hydrogénation, à éventuellement séparer l'ammoniac du milieu réactionnel,
- et à soumettre le milieu réactionnel à un ou plusieurs procédés de purification pour obtenir un lactame satisfaisant les spécifications désirées de pureté .

Le traitement d'hydrogénation réalisé directement sur le milieu réactionnel issu de l'hydrolyse cyclisante permet d'utiliser l'ammoniac produit pour mettre en oeuvre cette hydrogénation dans des conditions économiques satisfaisantes notamment avec une durée de cycle du catalyseur compatible avec une exploitation industrielle économique. Si nécessaire, il est possible d'ajuster la concentration en ammoniac soit par addition d'ammoniac, ou évaporation partielle de l'ammoniac produit par la réaction d'hydrolyse.

En outre, elle a également pour effet de diminuer la dégradation de l'indice UV qui est observée pendant la séparation des fractions légères du milieu réactionnel, notamment pendant la distillation de l'ammoniac et éventuellement de l'eau ou du solvant présent dans le milieu.

Selon l'invention, le lactame récupéré après le traitement d'hydrogénation et éventuellement la séparation de l'ammoniac peut être soumis à différentes étapes de purification connues et décrites dans de nombreux brevets tels que, par exemple le brevet US 5 496 941, WO 98/05636,

Les traitements possibles sont l'oxydation, la distillation en milieu acide ou basique, l'extraction liquide/liquide, le traitement sur résines échangeuse d'ions, la cristallisation, par exemple.

Tous ces traitements sont données uniquement à titre d'exemple, ils peuvent être tous réalisés ou uniquement certains d'entre eux et dans un ordre quelconque.

D'autre avantages, détails de l'invention apparaîtront plus clairement au vu des exemples donnés ci-dessous uniquement à titre indicatif, sans aucun effet de limitation de l'invention.

### Exemple 1 comparatif

Dans un réacteur d'hydrogénation à 80°C sous 25 bar, chargé avec 5 % de Nickel de Raney comme catalyseur d'hydrogénation, on introduit en continu une solution aqueuse constituée de 60 % de caprolactame issu d'une hydrolyse cyclisante d'un 6-aminocapronitrile dont les conditions sont décrites dans la demande de brevet français 2 714 379.

Cette solution de caprolactame ne contient plus d'ammoniac, celui-ci ayant été distillé en sortie de l'étape d'hydrolyse cyclisante.

L'indice UV de cette solution mesurée est de 24 à 290 nm.

L'indice UV de la solution sortante de l'étape d'hydrogénation est contrôlée périodiquement pour déterminer l'efficacité de l'hydrogénation et donc l'activité du catalyseur.

Les résultats sont indiqués dans le tableau ci-dessous.

| Durée de fonctionnement | 24 h | 48 h | 72 h | 120 h | 192 h |
|---|---|---|---|---|---|
| Indice UV | 9,7 | 22 | 22 | 23 | 23 |

Les résultats montrent que le catalyseur a perdu son activité dès le 2ème jour de fonctionnement.

### Exemple 2

L'exemple 1 est reproduit mais en utilisant comme solution de caprolactame dans l'eau à laquelle de l'ammoniac a été ajouté pour obtenir une solution contenant 90 g/l d'ammoniac et 61 % de caprolactame.

La solution de caprolactame avant traitement présente un indice UV mesuré à 290 nm de 1,6.

Les résultats sont indiqués dans le tableau suivant.

| Durée de fonctionnement | 48 h | 120 h | 192 h |
|---|---|---|---|
| Indice UV | 0,8 | 0,8 | 0,8 |

Ces résultats montrent clairement l'effet de la présence d'ammoniac sur la conservation de l'activité du catalyseur. Ainsi, après huit jours de fonctionnement, l'indice UV de la solution résultante est identique à celui obtenu en début d'opération.
Le traitement d'hydrogénation conserve son efficacité.

### Exemple 3

La solution de caprolactame traité par hydrogénation selon les conditions de l'exemple 1 est le milieu réactionnel issu de la réaction du 6 aminocapronitrile avec de l'eau. Ce milieu a subi un refroidissement rapide à une température de 80°C en sortie de réacteur pour éviter la polycondensation du caprolactame. Le milieu contient 90 g/l d'ammoniac produit par la réaction. Ce milieu présente un indice UV à 290 nm de 1,8. Le milieu est directement soumis à une hydrogénation selon les conditions de l'exemple 1. Les résultats obtenus sont donnés dans le tableau ci-dessous.

| Durée de fonctionnement hydrogénation | 48 h | 120 h | 192 h |
|---|---|---|---|
| indice UV | 0,8 | 0,8 | 0,8 |

Par ailleurs, l'aminocapronitrile qui n'a pas été transformé en caprolactame est totalement hydrogéné en hexaméthylène diamine, même après 192 heures de fonctionnement du catalyseur d'hydrogénation.

## Revendications

1. Procédé de traitement d'un milieu liquide contenant au moins de l'ε-caprolactame consistant à soumettre ledit milieu à une hydrogénation en présence d'un catalyseur d'hydrogénation **caractérisé en ce que** le milieu comprend de l'ammoniac dissous.

2. Procédé selon la revendication 1, **caractérisé en ce que** le milieu comprend un solvant choisi dans le groupe comprenant les alcools ayant de 1 à 3 atomes de carbone, l'eau et les mélanges eau / alcool.

3. Procédé selon la revendication 1 ou 2, **caractérisé en ce que** le traitement d'hydrogénation est mis en oeuvre à une température inférieure à 150°C.

4. Procédé selon la revendication 3, **caractérisé en ce que** le traitement d'hydrogénation est mis en oeuvre à une température comprise entre 50 et 150° C, de préférence entre 70°C et 130°C.

5. Procédé selon l'une des revendications précédentes, **caractérisé en ce que** la concentration en ammoniac dissous est supérieur à 10 g/l.

6. Procédé selon l'une des revendications précédentes, **caractérisé en ce que** le catalyseur d'hydrogénation est choisi dans le groupe comprenant le fer, le nickel, le cobalt, le ruthénium, le rhodium, le palladium, l'osmium, l'iridium, le platine pris seul ou en combinaison.

7. Procédé selon la revendication 6, **caractérisé en ce que** le catalyseur est un catalyseur massique ou un catalyseur supporté.

8. Procédé selon l'une des revendications 6 ou 7, **caractérisé en ce que** le catalyseur comprend au moins un additif choisi dans le groupe comprenant le zirconium, le manganèse, le cuivre, le chrome, le titane, le molybdène, le tungstène, le fer et le zinc.

9. Procédé selon l'une des revendications 7 ou 8, **caractérisé en ce que** le support du catalyseur est choisi dans le groupe comprenant les charbons actifs, les alumines, les silices, les oxyde de titane, les oxydes de terres rares, les oxydes de zirconium, les oxydes de zinc, les silicates ou phosphates de magnésium et/ou d'aluminium, les phosphates de bore.

10. Procédé selon l'une des revendications 7 à 9, **caractérisé en ce que** la concentration en élément catalytique dans les catalyseurs supportés est comprise entre 0,01 % et 80 % en poids exprimé en élément métal par rapport au poids de catalyseur.

11. Procédé selon l'une des revendications précédentes **caractérisé en ce que** le lactame est obtenu par hydrolyse cyclisante de l' aminocapronitrile.

12. Procédé de purification de lactames obtenus par hydrolyse cyclisante d'un aminonitrile **caractérisé en ce qu'**il consiste à :
- convertir de l'amincaproonitrile en ^{ε}-caprolactame par réaction avec l'eau en phase vapeur et production d'ammoniac,
- refroidir le milieu réactionnel d'hydrolyse cyclisante à une température inférieure à 150°C,
- soumettre ledit milieu réactionnel refroidi contenant au moins partiellement l'ammoniac produit par la réaction à une hydrogénation en présence d'un catalyseur d'hydrogénation selon l'une des revendications 1 à 12,
- à éventuellement séparer l'ammoniac du milieu réactionnel,
- et éventuellement soumettre ledit milieu réactionnel à une ou plusieurs étapes de séparation et de purification du lactame pour obtenir un lactame satisfaisant les spécifications désirées de pureté.

13. Procédé selon la revendication 12, **caractérisé en ce que** les étapes de séparation ou de purification du lactame sont choisies dans le groupe comprenant la distillation, l'oxydation, traitement sur résines échangeuses d 'ions, la distillation en présence d'acide ou de base, la cristallisation, l'extraction liquide / liquide, une ou plusieurs de ces étapes étant mises en oeuvre selon un ordre quelconque.

## Claims

1. Process for the treatment of a liquid medium comprising at least ε-caprolactam which consists in subjecting the said medium to a hydrogenation in the presence of a hydrogenation catalyst, **characterized in that** the medium comprises dissolved ammonia.

2. Process according to claim 1, **characterized in that** the medium comprises a solvent chosen from the group consisting of alcohols having from 1 to 3 carbon atoms, water and water/alcohol mixtures.

3. Process according to claim 1 or 2, **characterized in that** the hydrogenation treatment is carried out at a temperature of less than 150°C.

4. Process according to claim 3, **characterized in that** the hydrogenation treatment is carried out at a temperature of between 50 and 150°C. preferably between 70°C and 130°C.

5. Process according to one of the preceding claims, **characterized in that** the concentration of dissolved ammonia is greater than 10 g/l.

6. Process according to one of the preceding claims, **characterized in that** the hydrogenation catalyst is chosen from the group consisting of iron, nickel, cobalt, ruthenium, rhodium, palladium, osmium, iridium and platinum, taken alone or in combination.

7. Process according to claim 6, **characterized in that** the catalyst is a bulk catalyst or a supported catalyst.

8. Process according to either of claims 6 and 7, **characterized in that** the catalyst comprises at least one additive chosen from the group consisting of zirconium, manganese, copper, chromium, titanium, molybdenum, tungsten, iron and zinc.

9. Process according to either of claims 7 and 8, **characterized in that** the support of the catalyst is chosen from the group consisting of active charcoals, aluminas, silicas, titanium oxide, rare earth metal oxides, zirconium oxides, zinc oxides, magnesium and/or aluminium silicates or phosphates, and boron phosphates.

10. Process according to one of claims 7 to 9, **characterized in that** the concentration of catalytic element in the supported catalysts is between 0.01% and 80% by weight, expressed as metal element, with respect to the weight of catalyst.

11. Process according to one of the preceding claims, **characterized in that** the lactam is obtained by cyclizing hydrolysis of aminocapronitrile.

12. Process for the purification of lactams obtained by cyclizing hydrolysis of an aminonitrile, **characterized in that** it consists in:
- converting aminocapronitrile to ε-caprolactam by reaction with water in the vapour phase and production of ammonia,
- cooling the cyclizing hydrolysis reaction mixture to a temperature of less than 150°C,
- subjecting the said cooled reaction mixture, at least partially comprising the ammonia produced by the reaction, to a hydrogenation in the presence of a hydrogenation catalyst according to one of claims 1 to 11,
- in optionally separating the ammonia from the reaction mixture,
- and optionally subjecting the said reaction mixture to one or more stages of separation and of purification of the lactam, in order to obtain a lactam which satisfies the desired purity specifications.

13. Process according to claim 12, **characterized in that** the stages of separation or of purification of the lactam are chosen from the group consisting of distillation, oxidation, treatment over ion-exchange resins, distillation in the presence of acid or of base, crystallization or liquid/liquid extraction, one or more of these stages being carried out in any order.

## Patentansprüche

1. Verfahren zur Behandlung eines flüssigen Mediums, das mindestens ε-Caprolactam umfaßt, darin bestehend, daß man das genannte Medium einer Hydrierung in Anwesenheit eines Hydrierungskatalysators unterzieht, **dadurch gekennzeichnet, daß** das Medium gelöstes Ammoniak umfaßt.

2. Verfahren nach Anspruch 1, **dadurch gekennzeichnet, daß** das Medium ein Lösungsmittel umfaßt, gewählt aus der Gruppe, die Alkohole mit 1 bis 3 Kohlenstoffatomen, Wasser und die Mischungen Wasser/Alkohol umfaßt.

3. Verfahren nach Anspruch 1 oder 2, **dadurch gekennzeichnet, daß** die Behandlung der Hydrierung bei einer Temperatur von unter 150 °C durchgeführt wird.

4. Verfahren nach Anspruch 3, **dadurch gekennzeichnet, daß** die Behandlung der Hydrierung bei einer Temperatur zwischen 50 °C und 150 °C, vorzugsweise zwischen 70 °C und 130 °C durchgeführt wird.

5. Verfahren nach einem der vorstehenden Ansprüche, **dadurch gekennzeichnet, daß** die Konzentration an gelöstem Ammoniak über 10 g/l liegt.

6. Verfahren nach einem der vorstehenden Ansprüche, **dadurch gekennzeichnet, daß** der Hydrierungskatalysator aus der Gruppe gewählt wird, die Eisen, Nickel, Cobalt, Ruthenium, Rhodium, Palladium, Osmium, Iridium oder Platin umfaßt, allein genommen oder in Kombination.

7. Verfahren nach Anspruch 6, **dadurch gekennzeichnet, daß** der Katalysator ein Massekatalysator oder ein Trägerkatalysator ist.

8. Verfahren nach einem der Ansprüche 6 oder 7, **dadurch gekennzeichnet, daß** der Katalysator mindestens einen Zusatzstoff umfaßt, gewählt aus der Gruppe, die Zirkonium, Mangan, Kupfer, Chrom, Titan, Molybdän, Wolfram, Eisen und Zink umfaßt.

9. Verfahren nach einem der Ansprüche 7 oder 8, **dadurch gekennzeichnet, daß** der Träger des Katalysators aus der Gruppe gewählt wird, die Aktivkohlen, Tonerden, Kieselerden, Titanoxide, Oxide der Seltenen Erden, Zirkoniumoxide, Zinkoxide, die Silicate oder Phosphate von Magnesium und/oder Aluminium und die Borphosphate umfaßt.

10. Verfahren nach einem der Ansprüche 7 bis 9, **dadurch gekennzeichnet, daß** die Konzentration an dem katalytischen Element in den Trägerkatalysatoren zwischen 0,01 Gew.-% und 80 Gew.-% liegt, ausgedrückt in Metall-Element und bezogen auf das Gewicht des Katalysators.

11. Verfahren nach einem der vorstehenden Ansprüche, **dadurch gekennzeichnet, daß** das Lactam durch cyclisierende Hydrolyse von Aminocapronitril erhalten wird.

12. Verfahren zur Reinigung von Lactamen, die durch cyclisierende Hydrolyse eines Aminonitrils erhalten wurden, **dadurch gekennzeichnet, daß** es besteht in
- Umwandlung des Aminocapronitrils in ε-Caprolactam durch Reaktion mit Wasser in der Dampfphase und Erzeugung von Ammoniak,
- Abkühlung des Reaktionsmediums aus der cyclisierenden Hydrolyse auf eine Temperatur von unter 150 °C,
- Unterziehen des genannten, gekühlten Reaktionsmediums, das mindestens teilweise durch die Reaktion erzeugtes Ammoniak enthält, einer Hydrierung in Anwesenheit eines Hydrierungskatalysators nach einem der Ansprüche 1 bis 12,
- gegebenenfalls Abtrennung des Ammoniaks aus dem Reaktionsmedium, und
- gegebenenfalls Unterziehen des genannten Reaktionsmediums einer oder mehreren Stufen zur Abtrennung und Reinigung des Lactams, um ein Lactam zu erhalten, das die gewünschten Reinheits-Spezifikationen gewährleistet.

13. Verfahren nach Anspruch 12, **dadurch gekennzeichnet, daß** die Stufen zur Abtrennung oder Reinigung des Lactams aus der Gruppe gewählt werden, die Destillation, Oxidation, Behandlung über Ionenaustauscherharze, Destillation in Anwesenheit von Säure oder Base, Kristallisation, Flüssig-Flüssig-Extraktion umfaßt, wobei eine oder mehrere dieser Stufen in irgendeiner Reihenfolge durchgeführt werden.
